# FASCICULE DE BREVET EUROPEEN

(11) **EP 3 268 351 B1**
(45) Date de publication et mention de la délivrance du brevet: **08.05.2019**
(21) Numéro de dépôt: 16709320.2
(22) Date de dépôt: 07.03.2016
(51) Int. Cl.: C07C 313/04

(54) **PROCEDE DE PREPARATION DE COMPOSES OXYSULFURES ET FLUORES**
VERFAHREN ZUR HERSTELLUNG VON OXYSULFID UND FLUORINIERTEN VERBINDUNGEN
PROCESS FOR PREPARING OXYSULPHIDE AND FLUORINATED COMPOUNDS

(30) Priorité: 09.03.2015 FR 1551933
(43) Date de publication de la demande: 17.01.2018
(73) Titulaire: Rhodia Operations, 75009 Paris (FR)
(72) Inventeur: REVELANT, Denis, 69740 Genas (FR); DAMBRIN, Valery, 69230 Saint Genis-Laval (FR); METZ, François, 69540 Irigny (FR); VERDIER, Stephan, 69008 Lyon (FR)
(74) Mandataire: Menville, Laure
(86) Numéro de dépôt international: PCT/EP2016/054731
(87) Numéro de publication internationale: WO 2016/142315

(56) Documents cités:
- WO-A1-2009/068533
- FR-A1- 2 564 829
- FR-A1- 2 660 923
- DATABASE CA [Online] CHEMICAL ABSTRACTS SERVICE, COLUMBUS, OHIO, US; LONG, ZHENGYU ET AL: "Preparation of 2,2,2-trifluoroethylsulfinate and its derivatives", XP002752583, extrait de STN Database accession no. 2000:355524 -& CN 1 203 910 A (SHANGHAI INST. OF ORGANIC CHEMISTRY, CHINESE ACADEMY OF SCIENCES, PEOP) 6 janvier 1999 (1999-01-06)

## Description

### DOMAINE TECHNIQUE DE L'INVENTION

La présente invention concerne la préparation de composés oxysulfurés et fluorés. Plus spécifiquement, cette invention concerne un procédé amélioré permettant la préparation d'acide fluorosulfinique, d'acide fluorosulfonique et de bisfluorosulfonimide et de leurs sels, ainsi que d'acides fluoroalcanesulfiniques, d'acide fluoroalcanesulfoniques et de bisfluoroalcanesulfonimides et de leurs sels.

### ETAT DE LA TECHNIQUE

Les acides perhalogénoalcanesulfoniques et plus particulièrement l'acide trifluorométhanesulfonique sont utilisés comme catalyseurs ou comme intermédiaires en synthèse organique.

Des procédés pour la synthèse de tels composés organiques oxysulfurés et fluorés, et notamment de l'acide perfluorométhanesulfinique sous forme salifiée, sont déjà connus. Par exemple, le document WO 2007/128893 décrit la réaction dans un solvant organique polaire d'un acide fluorocarboxylique au moins partiellement salifié et d'un oxyde de soufre. Cette réaction présente toutefois l'inconvénient d'être incomplète. Il est nécessaire de prévoir une récupération et un recyclage de l'acide fluorocarboxylique non consommé, ce qui rend le procédé plus complexe.

Une autre voie consiste à faire réagir un halogénure de fluoroalkyle avec un oxyde de soufre. Par exemple, le document FR 2 564 829 décrit la préparation d'acide trifluorométhanesulfinique par réaction de l'halogénure de trifluorométhyle avec du SO₂ dans un solvant aprotique polaire, en présence d'un métal. Le document FR 2 660 923 décrit quant à lui la préparation de trifluorométhanesulfinate de sodium à partir de bromotrifluorométhane et de dioxyde de soufre. Dans ces procédés, on constate que les réactions sont conduites sous pression, en présence d'une grande quantité d'oxyde de soufre. Or la concentration élevée en oxyde de soufre favorise les réactions compétitives et consécutives et par conséquent la production d'impuretés ce qui affecte sensiblement le rendement de la réaction. Aussi, les procédés antérieurs sont contraints d'opérer en limitant le taux de conversion pour éviter une dégradation trop importante du produit désiré. De plus, les procédés de l'art antérieur sont mis en oeuvre dans des équipements spécifiques résistant à la pression.

C'est dans ce contexte que les inventeurs ont cherché à améliorer le procédé existant, et à proposer un procédé plus performant en termes de taux de conversion, de rendement et de productivité. Avantageusement, ce procédé amélioré permet de réduire les réactions consécutives, défavorables, ce qui permet d'atteindre une sélectivité élevée, par exemple d'au moins 70%, tout en convertissant quasi entièrement le produit de départ (taux de conversion atteignant par exemple au moins 90%), ce qui limite sensiblement les recyclages de produit de départ et simplifie considérablement l'aval du procédé. En outre, il est souhaitable de limiter la quantité utilisée d'oxyde de soufre, qui est un réactif toxique.

### BREVE DESCRIPTION DE L'INVENTION

L'invention a pour objet un procédé de préparation d'un composé oxysulfuré et fluoré comprenant la mise en réaction, en présence d'un solvant organique :
i) d'au moins un composé de formule Ea-X (I), où Ea représente un atome de fluor ou un groupe ayant de préférence de 1 à 10 atomes de carbone choisi parmi les fluoroalkyles, les perfluoroalkyles et les fluoroalkényles, et X représente le brome, le chlore ou l'iode, et
ii) d'un oxyde de soufre,
ledit procédé étant tel que la concentration en oxyde de soufre dissous dans le milieu réactionnel est maintenue à une valeur comprise entre 0,2% et 3% poids.

En outre, l'invention a aussi pour objet l'utilisation d'un composé oxysulfuré et fluoré ainsi obtenu pour la synthèse d'un composé sulfonimide de formule (Ea-SO₂)₂NH et de ses sels (Ea-SO₂)₂NM (M représentant un métal), d'un composé acide sulfonique fluoré de formule Ea-SO₂OH ou d'un composé anhydride sulfonique fluoré de formule (Ea-SO₂)₂O, Ea ayant la définition précisée plus haut dans la présente description.

### BREVE DESCRIPTION DE LA FIGURE

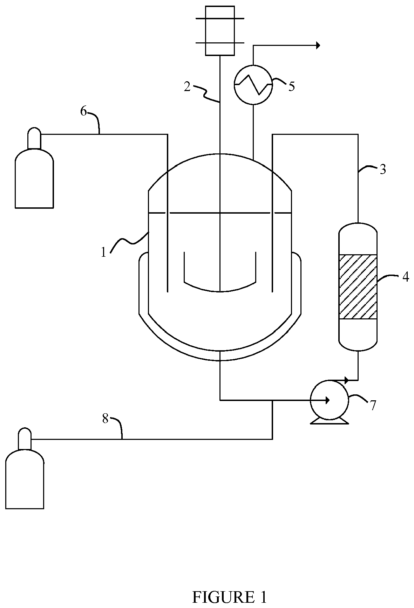

La figure 1 représente schématiquement le dispositif utilisé dans les exemples pour mettre en oeuvre le procédé objet de l'invention.

### DESCRIPTION DE L'INVENTION

Dans l'exposé qui suit, l'expression « compris entre ... et ... » doit être comprise comme incluant les bornes citées.

Conformément à l'invention, on entend par « milieu réactionnel » le milieu dans lequel a lieu la réaction chimique de sulfination. Le milieu réactionnel comprend le solvant de la réaction et, en fonction de l'avancement de la réaction, les réactifs et / ou les produits de la réaction. Il peut comprendre en outre des additifs et des impuretés.

Le procédé selon l'invention consiste en une réaction de sulfination entre au moins un composé de formule Ea-X (I) et un oxyde de soufre, et éventuellement une base et/ou un agent réducteur. Dans le composé de formule (I), Ea représente un atome de fluor ou un groupe choisi parmi les fluoroalkyles, les perfluoroalkyles et les fluoroalkényles, et X représente le brome, le chlore ou l'iode. Il est entendu que les définitions respectivement pour les groupes X et Ea peuvent être combinées.

Conformément à l'invention, on entend par fluoroalkyle un groupe formé d'une chaîne hydrocarbonée saturée linéaire ou ramifiée en C₁ à C₁₀ comportant au moins un atome de fluor. On entend par perfluoroalkyle un groupe formé d'une chaîne saturée linéaire ou ramifiée en C₁ à C₁₀ comprenant seulement des atomes de fluor, en plus des atomes de carbone, et dépourvue d'atome d'hydrogène. On entend par fluoroalkényle un groupe formé d'une chaîne hydrocarbonée en C₁ à C₁₀, linéaire ou ramifiée, comportant au moins un atome de fluor, et comprenant au moins une double liaison. Les groupes fluoroalkyles et fluoroalkényles peuvent éventuellement être substitués une ou plusieurs fois par un atome de brome, de chlore et/ou d'iode. Le groupe Ea peut ainsi représenter le radical CF₂Cl ou le radical CFCl₂.

Selon un mode de réalisation, Ea représente un atome de fluor. Le composé de formule (I) peut donc être un monofluorure de brome, de chlore ou d'iode.

Selon un autre mode de réalisation, le groupe Ea est choisi dans le groupe constitué par les fluoroalkyles ayant de 1 à 5 atomes de carbone, les perfluoroalkyles ayant de 1 à 5 atomes de carbone et les fluoroalkényles ayant de 1 à 5 atomes de carbone. De manière très préférée, le groupe Ea est choisi dans le groupe consistant en le radical CH₂F, le radical CHF₂, le radical C₂F₅ et le radical CF₃. De manière encore plus préférée, Ea représente le radical CF₃. Dans le composé de formule (I), X représente un atome halogène choisi parmi le brome, le chlore et l'iode, de préférence le brome. Le réactif de forme (I) est de préférence un halogénure de fluoroalkyle, de perfluoroalkyle ou de fluoroalkényle, et plus préférentiellement encore un bromure de fluoroalkyle, de perfluoroalkyle ou de fluoroalkényle. Le composé de formule (I) peut en particulier être choisi dans le groupe constitué par CF₃Br, CH₂FBr et CHF₂Br, et il s'agit tout particulièrement de CF₃Br.

Dans le procédé de l'invention, le composé de formule Ea-X (I) qu'on fait réagir peut être totalement ou en partie un composé recyclé, qui peut être obtenu par exemple par séparation à l'issue de la réaction du sulfination. Le composé de formule Ea-X (I) peut contenir des impuretés, par exemple des impuretés liées à son procédés d'obtention, par exemple des impuretés dihalogénées telles que CF₂Br₂, des traces de dihalogène telles que Cl₂ ou Br₂. En outre, le composé de formule Ea-X (I) peut provenir d'une autre unité industrielle utilisant en excès ou générant ce composé comme sous-produit. Il peut de ce fait contenir d'autres types d'impuretés, par exemple des impuretés soufrées.

Dans le procédé selon l'invention, ce composé (I) est mis en réaction avec un oxyde de soufre, et éventuellement au moins une base et/ou au moins un agent réducteur, en présence d'un solvant organique.

L'oxyde de soufre est de préférence le dioxyde de soufre. L'oxyde de soufre est généralement mis en oeuvre sous forme gazeuse. Il peut être également introduit sous forme de solution, dans le solvant organique choisi pour la réaction, à une concentration variant généralement entre 1% et 10% poids, de préférence entre 3% et 6% poids.

Il est également envisagé d'utiliser en tant qu'oxyde de soufre du trioxyde de soufre, également appelé anhydride sulfurique. Dans ce cas, le procédé selon l'invention consiste en une réaction de sulfonation, conduisant directement aux acides fluorosulfoniques et fluoroalcanesulfiniques. L'homme du métier est à même d'adapter les différentes étapes décrites dans cette description à cette variante de l'invention.

Selon un mode de réalisation du procédé selon l'invention, au moins une base est mise en réaction avec ledit composé de formule Ea-X (I) et l'oxyde de soufre. Une seule base ou un mélange de plusieurs bases peut être utilisé. La base peut être choisie parmi les bases minérales ou organiques. La base est choisie de manière à être soluble, au moins partiellement, dans le solvant organique de la réaction des sulfination. De manière préférée, ladite base est choisie parmi les hydroxydes, les carbonates et les alcoolates d'un cation monovalent ou divalent. Le cation monovalent ou divalent peut être choisi avantageusement dans le groupe constitué par les cations de métal alcalin, les cations de métal alcalinoterreux, les cations ammonium quaternaire et phosphonium quaternaire. De manière très préférée, il s'agit d'un cation alcalin, en particulier le sodium, le potassium, le césium et le rubidium, plus particulièrement le potassium. Comme cations ammonium ou phosphonium quaternaire, on peut citer les tétraalkylammonium ou phosphonium, trialkylbenzylammonium ou phosphonium, les tétraarylammonium ou phosphonium, dont les groupes alkyles identiques ou différents représentent une chaîne alkyle linéaire ou ramifiée ayant de 4 à 12 atomes de carbone, de préférence de 4 à 6 atomes de carbone et le groupe aryle est avantageusement un groupe phényle. On choisit préférentiellement le cation tétrabutylphosphonium. De façon préférée, la base peut être choisie dans le groupe constitué par l'hydroxyde de potassium et l'hydroxyde de sodium.

Selon un mode de réalisation du procédé selon l'invention, au moins un agent réducteur est mise en réaction avec ledit composé de formule Ea-X (I) et l'oxyde de soufre. Un seul agent réducteur ou un mélange de plusieurs agents réducteurs peut être utilisé. Tout type d'agent réducteur adapté au milieu réactionnel peut être utilisé. En particulier, l'agent réducteur peut être un composé choisi dans le groupe constitué par les formiates et les dithionites, de préférence les formiates et les dithionites d'un cation monovalent ou divalent. Le cation monovalent ou divalent peut être choisi avantageusement dans le groupe constitué par les cations de métal alcalin, les cations de métal alcalinoterreux, les cations ammonium quaternaire et phosphonium quaternaire. De manière très préférée, il s'agit d'un cation alcalin, en particulier le sodium, le potassium, le césium et le rubidium, plus particulièrement le potassium. Comme cations ammonium ou phosphonium quaternaire, on peut citer les tétraalkylammonium ou phosphonium, trialkylbenzylammonium ou phosphonium, les tétraarylammonium ou phosphonium, dont les groupes alkyles identiques ou différents représentent une chaîne alkyle linéaire ou ramifiée ayant de 4 à 12 atomes de carbone, de préférence de 4 à 6 atomes de carbone et le groupe aryle est avantageusement un groupe phényle. On choisit préférentiellement le cation tétrabutylphosphonium. De façon préférée, l'agent réducteur peut être le formiate de potassium ou le formiate de sodium. La présente invention n'exclue pas le fait que la base et l'agent réducteur soient le même composé. Ainsi, une base réductrice peut être mise en réaction avec ledit composé de formule Ea-X (I) et l'oxyde de soufre. Les formiates peuvent en particulier être considérés comme des bases réductrices. Dans le présent texte, les termes « base » et « agent réducteur » incluent les bases réductrices.

L'ajout d'un agent réducteur dans le milieu réactionnel n'est toutefois pas indispensable dans la présente invention car cet agent réducteur peut également être formé *in situ.* Par exemple :
- un solvant tel que le DMF peut réagir avec une base dans le milieu réactionnel et donner progressivement un agent réducteur tel qu'un formiate ;
- une base organique telle qu'une amine tertiaire, par exemple la N,N-diisopropyléthylamine, peut réagir avec l'oxyde de soufre etdonner progressivement un agent réducteur.

Le procédé selon l'invention est mis en oeuvre en présence d'un solvant organique, préférentiellement aprotique et très avantageusement aprotique polaire. De manière préférée, ledit solvant comporte très peu d'impuretés porteuses d'hydrogène acide. On entend par solvant aprotique, un solvant qui, dans la théorie de Lewis, n'a pas de protons à libérer. On fait appel, selon l'invention, à un solvant suffisamment stable dans les conditions réactionnelles. Il est souhaitable que le composé de formule (I) soit au moins partiellement soluble dans ce solvant. Ainsi, le solvant organique est choisi préférentiellement polaire. Il est ainsi préférable que le solvant aprotique polaire utilisable ait un moment dipolaire significatif. Ainsi, sa constante diélectrique relative ε est avantageusement au moins égale à environ 5. De préférence, sa constante diélectrique est inférieure ou égale à 50 et supérieure ou égale à 5, et est notamment comprise entre 30 et 40. Afin de déterminer si le solvant organique répond aux conditions de constante diélectrique énoncées ci-dessus, on peut se reporter, entre autres, aux tables de l'ouvrage Techniques of Chemistry, II - Organic solvents - 3ème édition (1970), p.536 et suivantes.

On préfère en outre que les solvants utilisés dans le procédé de l'invention soient susceptibles de bien solvater les cations, ce qui signifie que le solvant présente certaines propriétés de basicité au sens de Lewis. Pour déterminer si un solvant satisfait à cette exigence, on apprécie sa basicité en se référant au « nombre donneur ». On choisit un solvant organique polaire présentant un nombre donneur supérieur à 10, de préférence supérieur ou égal à 20. La borne supérieure ne présente aucun caractère critique. On choisit, de préférence, un solvant organique ayant un nombre donneur compris entre 10 et 30. On rappellera que le « nombre donneur » ou « donor number » désigné de manière abrégée DN, donne une indication sur le caractère nucléophile du solvant et révèle son aptitude à donner son doublet (voir par exemple la définition de DN dans l'ouvrage « Solvents and Solvent Effects in Organic Chemistry », C. Reichardt et T. Welton, 2011, .p.26-27).

Selon la présente invention, le ou les solvants polaires ne présentent pas d'hydrogène acide. En particulier lorsque le caractère polaire du ou des solvants est obtenu par la présence de groupes électro-attracteurs, il est souhaitable qu'il n'y ait pas d'hydrogène sur l'atome en position α de la fonction électro-attractrice. De manière plus générale, il est préférable que le pKa correspondant à la première acidité du solvant soit au moins égal à environ 20, avantageusement au moins égal à environ 25, de préférence compris entre 25 et 35. Le caractère acide peut également être exprimé par l'indice accepteur AN du solvant (voir par exemple la définition de AN dans l'ouvrage « Solvents and Solvent Effects in Organic Chemistry », C. Reichardt et T. Welton, 2011, .p.29-30). Avantageusement, cet indice accepteur AN est inférieur à 20, en particulier inférieur à 18.

Les solvants répondant aux différents impératifs et donnant de bons résultats peuvent être notamment des solvants de type amide. Parmi les amides, on comprend aussi les amides à caractère particulier comme les urées tétrasubstituées et les lactames monosubstitués. Les amides sont de préférence substitués (disubstitués pour les amides ordinaires). Le solvant organique peut être plus particulièrement choisi parmi le N,N-diméthylformamide (DMF), le N,N-diéthylformamide (DEF), le N,N-diméthylacétamide (DMAC), et les dérivés de pyrrolidone tels que la N-méthylpyrrolidone (NMP) et les mélanges de ceux-ci. Une autre catégorie particulièrement intéressante de solvant est constituée par les éthers, qu'ils soient symétriques ou non symétriques, qu'ils soient ouverts ou non. Dans la catégorie des éthers doivent être incorporés les différents dérivés des éthers de glycol tels que les différents glymes, le diglyme par exemple. Parmi les solvants précités, on met en oeuvre préférentiellement le DMF.

Selon des conditions préférées de mise en oeuvre du procédé de l'invention, il est souhaitable de contrôler la teneur en impuretés présentes dans le milieu réactionnel.

La teneur en atomes d'hydrogène labiles du système, ou plus exactement en protons libérables portés par ses divers composants, y compris leurs impuretés, est préférablement inférieure à la teneur en groupes fluorés libérés par la décomposition du composé de formule (I). Par atome d'hydrogène labile ou proton libérable, on entend un atome d'hydrogène qui est susceptible d'être arraché sous forme de proton par une base forte. En pratique, il s'agit des protons des fonctions acides qui présentent un pKa inférieur à environ 20. Plus la teneur en protons libérables sera faible, moins il y aura de risque de réaction parasite et meilleur sera le rendement. La teneur en protons libérables présents dans le milieu est au plus égale à 20% de la concentration molaire initiale dudit composé de formule (I). Avantageusement cette teneur est au plus égale à 10%, de préférence à 1% (en moles), par rapport à la teneur initiale en ledit composé de formule (I).

La principale molécule porteuse d'atomes d'hydrogène labiles est en général l'eau qui est susceptible de libérer jusqu'à deux protons par molécule. D'une manière générale il est préférable d'utiliser des réactifs et des solvants déshydratés, de manière que la teneur pondérale en eau de chacun des réactifs soit au plus égale à 1 pour 1000, par rapport à la masse totale dudit réactif. En fonction de l'ensemble des conditions réactionnelles, de telles teneurs en eau peuvent être satisfaisantes, mais dans certains cas, il peut être intéressant d'opérer à des niveaux plus bas, par exemple de l'ordre de 1 pour 10 000. Toutefois, il n'est pas nécessairement indispensable d'éliminer la totalité de l'eau et un rapport molaire eau/composé de formule (I) strictement inférieur à 10%, de préférence inférieur à 1% peut être toléré.

De plus, il est souhaitable que les impuretés métalliques soient en faibles quantités. Des éléments métalliques peuvent être présents en tant qu'impuretés apportées notamment par les réactifs, le solvant ou bien par les appareillages métalliques suite à une corrosion. Les deux catégories de métaux pouvant être essentiellement présents, à savoir les éléments de transition à deux états de valence (tels que le cuivre, le fer ou le chrome) et les éléments de la colonne VIII (notamment des métaux de la colonne du platine qui est le groupe constitué du platine, de l'osmium, de l'iridium, du palladium, du rhodium et du ruthénium), doivent être de préférence présents dans le milieu à une teneur exprimée par rapport au réactif (I) au plus égale à 1000 ppm molaires, de préférence à 10 ppm molaires.

Dans le procédé selon l'invention, la concentration en oxyde de soufre dissous dans le milieu réactionnel est maintenue à une valeur comprise entre 0,2% et 3% poids. De préférence, à l'intérieur de cette plage de valeur, cette concentration est maintenue constante. Par concentration constante, il convient d'entendre au sens de la présente invention, que ladite concentration peut varier de ± 20%, de préférence de ± 10%. La concentration en oxyde de soufre dissous dans le milieu réactionnel est de préférence maintenue constante durant toute la durée de la réaction. Le maintien d'une concentration constante peut être mis en oeuvre par un ajout continu d'oxyde de soufre au milieu réactionnel.

Conformément au procédé de l'invention, le suivi de la concentration en oxyde de soufre dissous, préférentiellement en dioxyde de soufre dissous, dans le milieu réactionnel peut être assuré par une méthode analytique. L'analyse en ligne ou in situ du milieu réactionnel est un moyen qui permet de maintenir constante la concentration en oxyde de soufre dissous. Le maintien d'une concentration constante peut être mis en oeuvre par un ajout continu et contrôlé d'oxyde de soufre au milieu réactionnel. Cet ajout contrôlé d'oxyde de soufre au milieu réactionnel, ajusté selon les résultats fournis par l'analyse en ligne ou in situ, permet de manière avantageuse de convertir le composé de formule (I) en un composé oxysulfuré et fluoré tout en défavorisant sensiblement la chimie parasite liée à la dégradation du produit de la réaction par l'oxyde de soufre, préférentiellement le dioxyde de soufre.

Conformément au procédé de l'invention, toute méthode analytique permettant de mesurer en ligne (via boucle d'échantillonnage) ou in situ la concentration en oxyde de soufre dissous, préférentiellement en dioxyde de soufre dissous, dans le milieu réactionnel comprenant au moins un solvant organique et un composé de formule (I) convient. De manière préférée, la concentration en oxyde de soufre dissous, préférentiellement en dioxyde de soufre dissous, dans le milieu réactionnel est suivie en ligne ou in situ par spectrométrie RAMAN, par spectroscopie proche infrarouge ou encore par spectroscopie UV. Par exemple, lorsque la concentration en oxyde de soufre dissous dans le milieu réactionnel est suivie par spectrométrie RAMAN, le réacteur est équipé d'une sonde Raman laquelle est reliée par une fibre optique au spectromètre Raman, ladite sonde permettant de suivre dans le milieu la concentration d'oxyde de soufre dissous.

Conformément au procédé de l'invention, on met en contact le composé de formule (I), l'oxyde de soufre et le solvant organique, et éventuellement la base et/ou l'agent réducteur. Le composé de formule (I) et l'oxyde de soufre peuvent être gazeux. Un moyen d'injection adéquat peut être alors utilisé pour favoriser la solubilisation de ces réactifs dans le solvant organique. De préférence, les réactifs sont injectés de façon à être dans un état de micromélange. Pour cela, tout dispositif connu par l'homme du métier pour favoriser la dispersion d'un réactif gazeux dans un milieu liquide peut être employé. Le ou les réactifs gazeux peuvent être introduits à différents endroits de l'appareillage, l'arrivée pouvant être faite dans le ciel du réacteur ou dans la masse réactionnelle. Par exemple, un tube plongeant muni préférentiellement d'une buse de dispersion peut être utilisé pour injecter un réactif dans un réacteur contenant le solvant organique. Selon un autre mode de réalisation, l'étape de mise en contact comprend l'introduction en ligne d'un réactif gazeux dans une boucle de circulation ou d'alimentation du solvant organique. Cette introduction peut être faite à l'aspiration ou au refoulement de la pompe de circulation. Selon un autre mode de réalisation, le ou les réactifs gazeux peuvent avoir été préalablement dissous dans un solvant organique, de préférence, celui de la réaction. Le réacteur contenant le solvant organique peut de préférence être un réacteur agité.

La mise en oeuvre peut être effectuée de manière semi-continue (ou semi-batch) ou d'une manière continue. De manière préférée, elle est effectuée de manière semi-continue.

Selon un mode de mise en oeuvre semi-continu, on introduit d'abord dans le solvant organique la base et/ou l'agent réducteur lorsqu'ils sont présents et on dissout une quantité initiale contrôlée d'oxyde de soufre. On peut alors introduire intégralement le composé de formule (I) dans le solvant organique puis ajouter en continu l'oxyde de soufre.

Selon un mode de réalisation en continu, l'ensemble des réactifs est introduit en continu. On alimente généralement le dispositif où se produit la réaction par le solvant organique éventuellement en mélange avec la base et/ou l'agent réducteur, et l'on introduit l'oxyde de soufre et le composé de formule (I).

Qu'il soit opéré en mode semi-continu ou continu, le procédé selon l'invention comprend de préférence le contrôle en ligne ou in situ de la concentration en oxyde de soufre dissous durant toute la durée de la réaction de manière à maintenir constante ladite concentration, dans le milieu réactionnel, dans une gamme comprise entre 0,2% et 3% poids.

Le procédé selon l'invention est avantageusement opéré dans un appareillage permettant une mise en oeuvre en semi-continu ou en continu. En particulier, un réacteur parfaitement agité, une cascade de réacteurs parfaitement agités avantageusement équipés d'une double enveloppe ou un réacteur tubulaire équipé d'une double enveloppe dans lequel circule un fluide caloporteur dont les caractéristiques permettent d'atteindre la température réactionnelle souhaitée conviennent à la mise en oeuvre du procédé selon l'invention.

Selon un mode de réalisation préféré du procédé selon l'invention, de la silice est introduite dans le milieu réactionnel, préférentiellement dans une quantité telle qu'elle représente de 0,1% à 10% poids, de préférence de 0,5% à 10% poids dans le milieu réactionnel. De manière particulière, la silice est ajoutée dans la solution formée du solvant organique et du composé de formule (I) lorsque le procédé selon l'invention est mis en oeuvre en semi-continu. L'ajout de silice permet de diminuer sensiblement l'impact corrosif sur le réacteur des fluorures générés dans le milieu par la mise en oeuvre du procédé selon l'invention.

Conformément au procédé de l'invention, le chauffage du mélange réactionnel a lieu avantageusement à une température comprise entre 40°C et 200°C, de préférence entre 50°C et 150°C. La réaction de sulfination selon l'invention peut être mise en oeuvre sous pression atmosphérique, à une pression supérieure à la pression atmosphérique ou à une pression inférieure à la pression atmosphérique. Ainsi, la pression totale absolue peut être choisie entre 1 bar et 20 bar et de préférence entre 1 bar et 3 bar. Selon un autre mode de réalisation, la pression totale absolue peut être comprise entre 1 mbar et 999 mbar, de manière préférée entre 500 mbar et 950 mbar, et de manière plus préférée entre 800 mbar et 900 mbar. De manière très préférée, la réaction est mise en oeuvre à pression atmosphérique.

La durée du chauffage peut varier largement en fonction de la température de réaction choisie. Elle peut varier entre environ 30 min à au plus une journée. Elle est avantageusement de plus d'une heure à moins de 20 heures et plus préférentiellement comprise entre 2 heures et 7 heures. Selon le mode de réalisation en continu, le temps de séjour moyen qui est défini comme le rapport entre le volume de la masse réactionnelle et le débit d'alimentation, se situe entre 30 min et 10 heures, et plus préférentiellement entre 2 heures et 4 heures.

Quel que soit le mode de mise en oeuvre de la réaction de sulfination, les réactifs sont de préférence introduits en quantité suffisante pour que le ratio global molaire de l'oxyde de soufre sur le composé de formule Ea-X (I) soit d'au moins 1. Si la réaction est conduite en présence d'une base et/ou d'un agent réducteur, les réactifs sont de préférence introduits en quantité suffisante pour que le ratio global molaire de l'oxyde de soufre sur le composé de formule Ea-X (I) soit d'au moins 2, quel que soit le mode de mise en oeuvre de la réaction de sulfination. De façon plus préférée, le ratio global molaire de l'oxyde de soufre sur le composé de formule Ea-X (I) est compris entre 2 et 5. En outre, les réactifs sont de préférence introduits en quantité suffisante pour que le ratio global molaire de base et/ou d'un agent réducteur sur le composé de formule Ea-X (I) soit d'au moins 3. De façon plus préférée, le ratio global molaire de base et/ou d'un agent réducteur sur le composé de formule Ea-X (I) est compris entre 3 et 6.

Lorsque la réaction de sulfination est conduite en présence d'une base et/ou d'un agent réducteur comprenant un cation monovalent ou divalent R, la mise en réaction d'un composé de formule Ea-X (I) avec un oxyde de soufre conduit à la préparation de composés de formule Ea-SOOR (III), où Ea a la même signification que dans la formule (I) et R représente un cation monovalent ou divalent. R peut être choisi avantageusement dans le groupe constitué par les cations de métal alcalin, les cations de métal alcalinoterreux, les cations ammonium quaternaire et phosphonium quaternaire. De manière très préférée, il s'agit d'un cation alcalin, en particulier le sodium, le potassium, le césium et le rubidium, plus particulièrement le potassium et le sodium, et encore plus particulièrement le potassium. Le procédé selon l'invention permet avantageusement de préparer un composé oxysulfuré et fluoré choisi dans le groupe constitué par F-SO₂R, CH₂F-SO₂R, CHF₂-SO₂R et CF₃-SO₂R, où R est défini comme ci-dessus, préférentiellement R est un cation alcalin, très préférentiellement le potassium. En particulier, le procédé selon la présente invention est un procédé de préparation d'un sel de l'acide fluorosulfinique, d'un sel de l'acide monofluorométhanesulfinique, d'un sel de l'acide difluorométhanesulfinique ou d'un sel de l'acide trifluorométhanesulfinique. Ainsi, le procédé selon l'invention permet avantageusement de préparer les composés F-SO₂K, CH₂F-SO₂K, CHF₂-SO₂K et CF₃-SO₂K.

A l'issue de la réaction de sulfination, le mélange résultant peut comprendre deux phases : une phase liquide, où une partie au moins du réactif (I) non consommé et du dioxyde de soufre sont dissous dans le solvant organique et une phase gazeuse contenant du dioxyde de soufre et éventuellement des impuretés légères formées lors de la réaction et des traces de composé de formule Ea-X (I) non converti.

L'avancement de la réaction peut être contrôlé par le taux de conversion (TT) du composé de formule (I) qui est le rapport molaire de la quantité de composé de formule (I) disparu sur la quantité de composé de formule (I) initiale dans le milieu réactionnel, ce taux étant calculé après dosage dudit composé de formule (I) restant dans le milieu.

Une fois le taux de transformation désiré atteint, le mélange réactionnel peut être traité de façon connue en soi pour séparer le produit obtenu, les produits de départ pouvant être recyclés pour produire une quantité supplémentaire du composé oxysulfuré et fluoré visé. Une ou plusieurs opérations de séparation liquide-solide peuvent être mises en oeuvre, par exemple pour séparer d'éventuelles impuretés solides du milieu réactionnel. Les techniques utilisées peuvent être la filtration sur différents types de supports, la centrifugation, la décantation et l'évaporation, cette liste n'étant pas exhaustive. Alternativement ou en plus, une ou des opérations de séparation liquide-liquide peuvent être mises en oeuvre pour séparer et/ou purifier le produit obtenu. Les techniques utilisées peuvent être la distillation, l'extraction liquide-liquide, la séparation par osmose inverse ou la séparation par des résines échangeuses d'ions, cette liste n'étant pas exhaustive. Ces opérations de séparation liquide-solide et liquide-liquide peuvent être conduites en régime continu ou discontinu, l'homme du métier pouvant choisir le régime le plus approprié.

Selon un mode réalisation, le procédé comprend en outre une étape postérieure à la réaction de sulfination consistant à séparer le composé de formule (I) n'ayant pas réagi et à recycler ce composé dans le procédé.

Les sous-produits de cette réaction peuvent être valorisés. Les halogénures peuvent notamment être récupérés et valorisés sous différentes formes, par exemple en régénérant le dihalogène ou en générant l'halogénure d'hydrogène. Les effluents du procédé sont de préférence traités de façon à éliminer les halogènes pouvant être présents.

Le composé oxysulfuré et fluoré préparé selon le procédé de l'invention peut avantageusement être utilisé pour la synthèse d'un composé sulfonimide de formule (Ea-SO₂)₂NH et de ses sels (Ea-SO₂)₂NM (M représentant un métal), d'un composé acide sulfonique fluoré de formule Ea-SO₂OH ou d'un composé anhydride sulfonique fluoré de formule (Ea-SO₂)₂O, Ea ayant la définition précisée plus haut dans la présente description. C'est pourquoi la présente invention a également pour objet un procédé de préparation d'un composé choisi dans le groupe constitué par un composé sulfonimide de formule (Ea-SO₂)₂NH et de ses sels (Ea-SO₂)₂NM (M représentant un métal), un composé acide sulfonique fluoré de formule Ea-SO₂OH et un composé anhydride sulfonique fluoré de formule (Ea-SO₂)₂O, Ea ayant la définition précisée plus haut dans la présente description, ledit procédé comprenant :
- une étape de préparation d'un composé oxysulfuré et fluoré selon le procédé décrit ci-avant,
- une étape dans laquelle ledit composé oxysulfuré et fluoré est utilisé en tant que composé réactif pour la synthèse d'un composé sulfonimide de formule (Ea-SO₂)₂NH et de ses sels (Ea-SO₂)₂NM (M représentant un métal), d'un composé acide sulfonique fluoré de formule Ea-SO₂OH ou d'un composé anhydride sulfonique fluoré de formule (Ea-SO₂)₂O, Ea ayant la définition précisée plus haut dans la présente description.

En premier lieu, la présente invention a pour objet un procédé de préparation d'un sel d'un composé sulfonimide de formule (Ea-SO₂)₂NM à partir d'un composé de formule Ea-SOOR (III) comprenant :
(a1) la préparation d'un composé de formule Ea-SOOR (III) selon le procédé décrit ci-avant,
(b1) la bromation, chloration ou fluoration du composé de formule (III) pour former un composé de formule Ea-SO₂X', où X' représente le brome, le chlore ou le fluor,
(c1) l'ammonolyse du composé Ea-SO₂X' à l'aide d'une amine tertiaire NR"3 en (EaSO₂)₂NH.NR"₃, avec R", identiques ou différents, représentant un groupe alkyle, linéaire ou ramifié, ayant de 1 à 20 atomes de carbone,
(d1) l'acidification de (EaSO₂)₂NH.NR"₃ pour obtenir le composé sulfonimide (Ea-SO₂)₂NH,
et éventuellement :
(e1) la neutralisation, par une base de métal M, en particulier par un hydroxyde alcalin, du composé (Ea-SO₂)₂NH pour former le sel de formule (Ea-SO₂)₂NM,
   et éventuellement
(f1) le séchage du sel (Ea-SO₂)₂NM.

De manière préférée, M est un métal choisi dans le groupe constitué par les métaux alcalins, par exemple Li et Na, les métaux alcalino-terreux, par exemple Mg, les terres-rares, par exemple La et les métaux de transition, par exemple Zn. De manière plus préférée, M est le lithium ou le sodium, et de manière encore plus préféré, M est le lithium. L'amine tertiaire mise en oeuvre pour l'étape (c1) d'ammonolyse peut être par exemple la di-isopropyl-éthyl-amine (EDIPA). Les étapes (c1), (d1), (e1) et (f1) sont connues de l'Homme du métier. En particulier, l'étape d'ammonolyse est décrite dans le brevet US 5 723 664. Les étapes de bromation, chloration ou fluoration, d'acidification, de neutralisation et de séchage sont des étapes classiques pouvant être effectuées dans les conditions connues de l'Homme du métier.

Selon un autre de ses aspects, la présente invention a également pour objet l'enchaînement des étapes (a1) et (b1) décrites ci-dessus. Ainsi, l'invention concerne un procédé de préparation d'un composé Ea-SO₂X', où X' représente le brome, le chlore ou le fluor, comprenant :
- la préparation d'un composé de formule Ea-SOOR (III) selon le procédé décrit ci-avant,
- la bromation, chloration ou fluoration du composé de formule (III) pour former un composé de formule Ea-SO₂X', où X' représente le brome, le chlore ou le fluor.

De manière préférée, le composé obtenu par ce procédé selon l'invention est un composé sulfonimide ou un de ses sels choisi dans le groupe constitué par bis-(trifluorométhanesulfonyl)imide de formule (CF₃SO₂)₂NH, du bis-(trifluorométhane-sulfonyl)imidure de lithium de formule (CF₃SO₂)2NLi (couramment noté LiTFSI), du bis-(fluorosulfonyl)imide de formule (F-SO₂)₂NH et du bis-(fluorosulfonyl)imidure de lithium de formule (F-SO₂)₂NLi (couramment noté LiFSI).

Les composés sulfonimides et leurs sels préparés selon les procédés décrits ci-dessus peuvent avantageusement être utilisés comme sels d'électrolyte, comme précurseurs d'agent antistatique ou encore comme précurseurs de tensio-actif. En particulier, lesdits composés peuvent avantageusement être employés comme électrolytes pour la fabrication de batteries, dans le domaine de l'électrochromisme, de l'électronique et de l'électrochimie. Ils sont avantageusement employés comme agents antistatiques pour la fabrication d'adhésifs sensibles à la pression (PSA : pressure sensitive adhesives). En tant qu'agent antistatique, ils peuvent encore être employés comme composants de lubrifiants. Ils sont utilisés dans les matériaux optiques tels que les appareils électroluminescents et entrent dans la composition de panneaux photovoltaïques. Ces utilisations sont également des objets de l'invention. En particulier, l'invention a pour objet un procédé de fabrication d'un dispositif électrochimique, de préférence une batterie, ledit procédé comprenant une étape de préparation d'un composé sulfonimide ou de ses sels selon le procédé décrit ci-avant, et une étape de fabrication du dispositif électrochimique dans lequel le composé sulfonimide ou ses sels est employé comme électrolyte.

Le composé oxysulfuré et fluoré préparé selon le procédé de l'invention est encore avantageusement utilisé pour la préparation, par oxydation, d'un composé acide sulfonique fluoré de formule Ea-SO₂-OH où Ea est défini comme ci-dessus. A cet effet, on soumet par exemple ledit composé oxysulfuré et fluoré issu du procédé de l'invention à une étape d'acidification pour libérer le composé Ea-SO₂-OH, par exemple en utilisant une solution d'un acide minéral fort, tel que l'acide sulfurique ou l'acide chlorhydrique. La présente invention a donc également pour objet un procédé de préparation d'un composé acide sulfonique fluoré de formule Ea-SO₂-OH à partir d'un composé oxysulfuré et fluoré comprenant :
(a2) la préparation d'un composé de formule Ea-SOOR (III) selon le procédé décrit ci-avant,
(b2) l'oxydation dudit composé de formule Ea-SOOR (III) pour obtenir un composé acide sulfonique fluoré de formule Ea-SO₂-OH, où Ea est défini comme ci-dessus.

De manière préférée, le composé de formule Ea-SOOR (III) est un sel de métal alcalin de trifluorométhylsulfinate (CF₃SO₂R où R est un métal alcalin) de manière à pouvoir l'utiliser dans la synthèse de l'acide trifluorométhanesulfonique (appelé acide triflique) de formule CF₃SO₂OH.

Le composé Ea-SO₂-OH ainsi obtenu peut avantageusement être transformé en anhydride sulfonique fluoré de formule (Ea-SO₂)₂O. La réaction d'anhydrisation est connue de l'Homme du métier et est en particulier décrite dans le brevet US 8 222 450. La présente invention a également pour objet un procédé de préparation d'un composé anhydride sulfonique fluoré de formule (Ea-SO₂)₂O, où Ea est défini comme ci-dessus, à partir d'un composé oxysulfuré et fluoré comprenant :
(a2) la préparation d'un composé de formule Ea-SOOR (III) selon le procédé décrit ci-avant,
(b2) l'oxydation dudit composé de formule Ea-SOOR (III) pour obtenir un composé acide sulfonique fluoré de formule Ea-SO₂-OH,
(c2) l'anhydrisation du composé acide sulfonique fluoré de formule Ea-SO₂-OH pour obtenir un composé anhydride sulfonique fluoré de formule (Ea-SO₂)₂O.

De manière préférée, le composé de formule Ea-SOOR (III) est un sel de métal alcalin de trifluorométhylsulfinate de manière à ce que l'anhydrisation de l'acide triflique conduise à la production de l'anhydride trifluorométhanesulfonique de formule (CF₃SO₂)₂O.

De manière préférée, le composé obtenu par ce procédé selon l'invention est un acide sulfonique fluoré ou un composé anhydride sulfonique fluoré choisi dans le groupe constitué par l'acide trifluorométhanesulfonique de formule CF₃SO₂OH et l'anhydride trifluorométhanesulfonique de formule (CF₃SO₂)₂O.

Les composé acides sulfoniques fluorés de formule Ea-SO₂-OH et les composés anhydrides sulfoniques fluorés de formule (Ea-SO₂)₂O peuvent être utilisés dans diverses applications, notamment comme catalyseur acide, comme groupe de protection en synthèse organique, comme synthon dans les domaines de la pharmaceutique, l'agrochimie ou l'électronique, ou comme sel pour l'électronique.

Les exemples qui suivent, accompagnés de la Figure 1, illustrent l'invention sans toutefois la limiter.

### EXEMPLES

### Exemple 1 : préparation de trifluorométhylsulfinate de potassium CF₃SO₂K par sulfination de bromotrifluorométhane en présence de SO₂ et d'hydroxyde de potassium selon une mise en oeuvre en continu

Le dispositif représenté sur la figure 1 est utilisé.

Dans un réacteur (1) de 500 ml muni d'une agitation (2), d'une boucle de circulation (3) équipée d'un mélangeur statique (4) et surmonté d'un réfrigérant (5), on introduit 300 g de DMF et 33,6 g (0,6 moles) d'hydroxyde de potassium en perles. On chauffe à 70°C et on introduit par un tube plongeant (6) du dioxyde de souffre gazeux de manière à obtenir une concentration dans le DMF de 1% poids. Ce niveau de concentration est vérifié par dosage Raman in situ. Lorsque cette concentration est stabilisée, on introduit à l'aspiration de la pompe de la boucle de circulation (7) le CF₃Br gazeux par la voie (8) à un débit de 10 g/h. Pendant cette phase d'introduction du CF₃Br, la concentration en SO₂ dans le réacteur est maintenue à 1% par un appoint continu de gaz et à l'aide du dosage Raman in-situ. La phase d'introduction de CF₃Br a une durée de 3 heures, soit 30 g (0,2 moles) de CF₃Br introduits au total. On maintient le milieu à 70°C pendant 30 minutes après la fin d'introduction du CF₃Br, puis on refroidi le mélange à température ambiante.

On dose dans le brut réactionnel final 31 g de CF₃SO₂K (0,18 moles) soit un rendement de 90% en CF₃SO₂K par rapport au CF₃Br introduit.

### Exemple 2 (comparatif) :

Le procédé de préparation de CF₃Br décrit dans l'exemple 1 a été reproduit, mais la réaction a été conduite sous une pression de 5 bar avec une régulation de la concentration de SO₂ dans le DMF de 10% poids. Après dosage dans le brut réactionnel final, le rendement en CF₃SO₂K par rapport au CF₃Br introduit est de 33%.

### Exemple 3 : préparation de trifluorométhylsulfinate de sodium CF₃SO₂Na par sulfination de bromotrifluorométhane en présence de SO₂ et de formiate de sodium selon une mise en oeuvre en continu

Le dispositif représenté sur la figure 1 est utilisé.

Dans un réacteur (1) de 500 ml muni d'une agitation (2), d'une boucle de circulation (3) équipée d'un mélangeur statique (4) et surmonté d'un réfrigérant (5), on introduit 300 g de DMF et 15 g (0,22 moles) de poudre de formiate de sodium. On chauffe à 70°C et on introduit par un tube plongeant (6) du dioxyde de souffre gazeux de manière à obtenir une concentration dans le DMF de 1% poids. Ce niveau de concentration est vérifié par dosage Raman in situ. Lorsque cette concentration est stabilisée, on introduit à l'aspiration de la pompe de la boucle de circulation (7) le CF₃Br gazeux par la voie (8) à un débit de 10 g/h. Pendant cette phase d'introduction du CF₃Br, la concentration en SO₂ dans le réacteur est maintenue à 1% par un appoint continu de gaz et à l'aide du dosage Raman in-situ. La phase d'introduction de CF₃Br a une durée de 3 heures, soit 30 g (0,2 moles) de CF₃Br introduits au total. On maintient le milieu à 70°C pendant 30 minutes après la fin d'introduction du CF₃Br, puis on refroidi le mélange à température ambiante.

On dose dans le brut réactionnel final 31 g de CF₃SO₂Na (0,18 moles) soit un rendement de 90% en CF₃SO₂Na par rapport au CF₃Br introduit.

## Revendications

1. Procédé de préparation d'un composé oxysulfuré et fluoré comprenant la mise en réaction, en présence d'un solvant organique :
i) d'au moins un composé de formule Ea-X (I), où Ea représente un atome de fluor ou un groupe ayant de préférence de 1 à 10 atomes de carbone, choisi parmi les fluoroalkyles, les perfluoroalkyles et les fluoroalkényles, et X représente le brome, le chlore ou l'iode, et
ii) d'un oxyde de soufre,
ledit procédé étant tel que la concentration en oxyde de soufre dissous dans le milieu réactionnel est maintenue à une valeur comprise entre 0,2% et 3% poids.

2. Procédé selon la revendication 1, **caractérisé en ce que** le composé de formule (I) est choisi dans le groupe constitué par le mono fluorure de brome, de chlore ou d'iode et un halogénure de fluoroalkyle, de perfluoroalkyle ou de fluoroalkényle, de façon préférée dans le groupe constitué par CF₃Br, CH₂FBr et CHF₂Br, et de façon plus préférée il s'agit de CF₃Br.

3. Procédé selon la revendication 1 ou la revendication 2, **caractérisé en ce que** le solvant organique est choisi parmi le N,N-diméthylformamide (DMF), le N,N-diéthylformamide (DEF), le N,N-diméthylacétamide (DMAC), et les dérivés de pyrrolidone tels que la N-méthylpyrrolidone (NMP) et les mélanges de ceux-ci, et de façon préférée le solvant est le DMF.

4. Procédé selon l'une quelconque des revendications 1 à 3, **caractérisé en ce que** la concentration en oxyde de soufre dissous dans le milieu réactionnel est suivie en ligne ou in situ par spectrométrie RAMAN, par spectroscopie proche infrarouge ou par spectroscopie UV.

5. Procédé selon l'une quelconque des revendications 1 à 4, **caractérisée en ce qu'**au moins une base est mise en réaction avec ledit composé de formule Ea-X (I) et ledit oxyde de soufre, la base étant choisie de préférence parmi les hydroxydes, les carbonates et les alcoolates d'un cation monovalent ou divalent choisi avantageusement dans le groupe constitué par les cations de métal alcalin, les cations de métal alcalinoterreux, les cations ammonium quaternaire et phosphonium quaternaire, et de façon préférée la base est choisie dans le groupe constitué par l'hydroxyde de potassium et l'hydroxyde de sodium.

6. Procédé selon l'une quelconque des revendications 1 à 5, **caractérisée en ce qu'**au moins un agent réducteur est mise en réaction avec ledit composé de formule Ea-X (I) et ledit oxyde de soufre, l'agent réducteur étant choisi de préférence dans le groupe constitué par les formiates et les dithionites d'un cation monovalent ou divalent choisi avantageusement dans le groupe constitué par les cations de métal alcalin, les cations de métal alcalinoterreux, les cations ammonium quaternaire et phosphonium quaternaire, et de façon préférée l'agent réducteur est choisi dans le groupe constitué par le formiate de potassium et le formiate de sodium.

7. Procédé selon la revendication 5 ou la revendication 6, caractérisé en ce le composé oxysulfuré et fluoré obtenu est choisi dans le groupe constitué par F-SO₂R, CH₂F-SO₂R, CHF₂-SO₂R et CF₃-SO₂R, où R est cation monovalent ou divalent, préférentiellement R est un cation alcalin, très préférentiellement le potassium.

8. Procédé de préparation d'un composé choisi dans le groupe constitué par un composé sulfonimide de formule (Ea-SO₂)₂NH et de ses sels (Ea-SO₂)₂NM, un composé acide sulfonique fluoré de formule Ea-SO₂OH et un composé anhydride sulfonique fluoré de formule (Ea-SO₂)₂O, Ea ayant la définition précisée dans les revendications 1 à 7 et M représentant un métal, ledit procédé comprenant :
- une étape de préparation d'un composé oxysulfuré et fluoré selon le procédé décrit ci-avant dans l'une quelconque des revendications 1 à 7,
- une étape dans laquelle ledit composé oxysulfuré et fluoré est utilisé en tant que composé réactif pour la synthèse d'un composé sulfonimide de formule (Ea-SO₂)₂NH et de ses sels (Ea-SO₂)₂NM, d'un composé acide sulfonique fluoré de formule Ea-SO₂OH ou d'un composé anhydride sulfonique fluoré de formule (Ea-SO₂)₂O.

9. Procédé selon la revendication 8, **caractérisé en ce que** le composé obtenu est un composé sulfonimide ou un de ses sels choisi dans le groupe constitué par le bis-(trifluorométhanesulfonyl)imide de formule (CF₃SO₂)₂NH, le bis-(trifluorométhane-sulfonyl)imidure de lithium de formule (CF₃SO₂)₂NLi, le bis-(fluorosulfonyl)imide de formule (F-SO₂)₂NH et le bis-(fluorosulfonyl)imidure de lithium de formule (F-SO₂)₂NLi.

10. Procédé selon la revendication 8, **caractérisé en ce que** le composé obtenu est un acide sulfonique fluoré ou un composé anhydride sulfonique fluoré choisi dans le groupe constitué par l'acide trifluorométhanesulfonique de formule CF₃SO₂OH et l'anhydride trifluorométhanesulfonique de formule (CF₃SO₂)₂O.

## Patentansprüche

1. Verfahren zur Herstellung einer Fluoroxysulfid-Verbindung, bei dem man in Gegenwart eines organischen Lösungsmittels:
i) mindestens eine Verbindung der Formel Ea-X (I), wobei Ea für ein Fluoratom oder eine Gruppe mit vorzugsweise 1 bis 10 Kohlenstoffatomen, die aus Fluoralkylgruppen, Perfluoralkylgruppen und Fluoralkenylgruppen ausgewählt ist, steht und X für Brom, Chlor oder Iod steht, und
ii) ein Schwefeloxid
umsetzt, wobei das Verfahren so beschaffen ist, dass die Konzentration von in dem Reaktionsmedium gelöstem Schwefeloxid bei einem Wert zwischen 0,2 und 3 Gew.-% gehalten wird.

2. Verfahren nach Anspruch 1, **dadurch gekennzeichnet, dass** die Verbindung der Formel (I) aus der Gruppe bestehend aus Brom-, Chlor- oder Iodmonofluorid und einem Fluoralkyl-, Perfluoralkyl- oder Fluoralkenylhalogenid und vorzugsweise aus der Gruppe bestehend aus CF₃Br, CH₂FBr und CHF₂Br ausgewählt wird und weiter bevorzugt CF₃Br ist.

3. Verfahren nach Anspruch 1 oder Anspruch 2, **dadurch gekennzeichnet, dass** das organische Lösungsmittel aus N,N-Dimethylformamid (DMF), N,N-Diethylformamid (DEF), N,N-Dimethylacetamid (DMAC) und Pyrrolidonderivaten, wie N-Methylpyrrolidon (NMP), und Mischungen davon ausgewählt wird und es sich bei dem Lösungsmittel vorzugsweise um DMF handelt.

4. Verfahren nach einem der Ansprüche 1 bis 3, **dadurch gekennzeichnet dass** die Konzentration von in dem Reaktionsmedium gelöstem Schwefeloxid inline oder in situ durch Raman-Spektrometrie, Nahinfrarot-Spektroskopie oder UV-Spektroskopie verfolgt wird.

5. Verfahren nach einer der Ansprüche 1 bis 4, **dadurch gekennzeichnet, dass** man mindestens eine Base mit der Verbindung der Formel Ea-X (I) und dem Schwefeloxid umsetzt, wobei die Base vorzugsweise aus Hydroxiden, Carbonaten und Alkoholaten eines vorteilhafterweise aus der Gruppe bestehend aus Alkalimetallkationen, Erdalkalimetallkationen, quartären Ammoniumkationen und quartären Phosphoniumkationen ausgewählten einwertigen oder zweiwertigen Kations ausgewählt wird und die Base vorzugsweise aus der Gruppe bestehend aus Kaliumhydroxid und Natriumhydroxid ausgewählt wird.

6. Verfahren nach einer der Ansprüche 1 bis 5, **dadurch gekennzeichnet, dass** man mindestens ein Reduktionsmittel mit der Verbindung der Formel Ea-X (I) und dem Schwefeloxid umsetzt, wobei das Reduktionsmittel vorzugsweise aus der Gruppe bestehend aus Formiaten und Dithioniten eines vorteilhafterweise aus der Gruppe bestehend aus Alkalimetallkationen, Erdalkalimetallkationen, quartären Ammoniumkationen und quartären Phosphoniumkationen ausgewählten einwertigen oder zweiwertigen Kations ausgewählt wird und das Reduktionsmittel vorzugsweise aus der Gruppe bestehend aus Kaliumformiat und Natriumformiat ausgewählt wird.

7. Verfahren nach Anspruch 5 oder Anspruch 6, **dadurch gekennzeichnet, dass** die erhaltene Fluoroxysulfid-Verbindung aus der Gruppe bestehend aus F-SO₂R, CH₂F-SO₂R, CHF₂-SO₂R and CF₃-SO₂R ausgewählt wird, wobei R für ein einwertiges oder zweiwertiges Kation steht und R vorzugsweise für ein Alkalikation, besonders bevorzugt Kalium, steht.

8. Verfahren zur Herstellung einer Verbindung aus der Gruppe bestehend aus einer Sulfonimidverbindung der Formel (Ea-SO₂)₂NH und ihren Salzen (Ea-SO₂)₂NM, einer Fluorsulfonsäureverbindung der Formel Ea-SO₂OH und einer Fluorsulfonsäureanhydridverbindung der Formel (Ea-SO₂)₂O, wobei Ea die in den Ansprüchen 1 bis 7 angegebene Definition aufweist und M für ein Metall steht, wobei das Verfahren Folgendes umfasst:
- einen Schritt der Herstellung einer Fluoroxysulfid-Verbindung gemäß dem oben in einem der Ansprüche 1 bis 7 beschriebenen Verfahren,
- einen Schritt, bei dem die Fluoroxysulfid-Verbindung als reaktive Verbindung für die Synthese einer Sulfonimidverbindung der Formel (Ea-SO₂)₂NH und deren Salzen (Ea-SO₂)₂NM, einer Fluorsulfonsäureverbindung der Formel Ea-SO₂OH und einer Fluorsulfonsäureanhydridverbindung der Formel (Ea-SO₂)₂O verwendet wird.

9. Verfahren nach Anspruch 8, **dadurch gekennzeichnet, dass** es sich bei der erhaltenen Verbindung um eine Sulfonimidverbindung oder eines ihrer Salze aus der Gruppe bestehend aus Bis(trifluormethansulfonyl)imid der Formel (CF₃SO₂)₂NH, Lithiumbis-(trifluormethansulfonyl)imid der Formel (CF₃SO₂)₂NLi, Bis(fluorsulfonyl)imid der Formel (F-SO₂)₂NH und Lithiumbis(fluorsulfonyl)imid der Formel (F-SO₂)₂NLi handelt.

10. Verfahren nach Anspruch 8, **dadurch gekennzeichnet, dass** es sich bei der erhaltenen Verbindung um eine Fluorsulfonsäure oder eine Fluorsulfonsäureanhydridverbindung aus der Gruppe bestehend aus Trifluormethansulfonsäure der Formel CF₃SO₂OH und Trifluormethansulfonsäureanhydrid der Formel (CF₃SO₂)₂O handelt.

## Claims

1. Process for the preparation of an oxysulfide and fluorinated compound, comprising the reaction, in the presence of an organic solvent:
i) of at least one compound of formula Ea-X (I), where Ea represents a fluorine atom or a group preferably having from 1 to 10 carbon atoms chosen from fluoroalkyls, perfluoroalkyls and fluoroalkenyls, and X represents bromine, chlorine or iodine, and
ii) of a sulfur oxide,
said process being such that the concentration of sulfur oxide dissolved in the reaction medium is maintained at a value of between 0.2 weight% and 3 weight%.

2. Process according to Claim 1, **characterized in that** the compound of formula (I) is selected from the group consisting of bromine, chlorine or iodine monofluoride and a fluoroalkyl, perfluoroalkyl or fluoroalkenyl halide, preferably from the group consisting of CF₃Br, CH₂FBr and CHF₂Br, and more preferably it is CF₃Br.

3. Process according to Claim 1 or Claim 2, **characterized in that** the organic solvent is chosen from N,N-dimethylformamide (DMF), N,N-diethylformamide (DEF), N,N-dimethylacetamide (DMAC) and pyrrolidone derivatives, such as N-methylpyrrolidone (NMP), and the mixtures of these, and preferably the solvent is DMF.

4. Process according to any one of Claims 1 to 3, **characterized in that** the concentration of dissolved sulfur oxide in the reaction medium is monitored in-line or in situ by Raman spectrometry, by near infrared spectroscopy or by UV spectroscopy.

5. Process according to any one of Claims 1 to 4, **characterized in that** at least one base is reacted with said compound of formula Ea-X (I) and said sulfur oxide, the base preferably being chosen from hydroxides, carbonates and alkoxides of a monovalent or divalent cation advantageously selected from the group consisting of alkali metal cations, alkaline earth metal cations, quaternary ammonium cations and quaternary phosphonium cations, and preferably the base is selected from the group consisting of potassium hydroxide and sodium hydroxide.

6. Process according to any one of Claims 1 to 5, **characterized in that** at least one reducing agent is reacted with said compound of formula Ea-X (I) and said sulfur oxide, the reducing agent preferably being selected from the group consisting of formates and dithionites of a monovalent or divalent cation advantageously selected from the group consisting of alkali metal cations, alkaline earth metal cations, quaternary ammonium cations and quaternary phosphonium cations, and preferably the reducing agent is selected from the group consisting of potassium formate and sodium formate.

7. Process according to Claim 5 or Claim 6, **characterized in that** the oxysulfide and fluorinated compound obtained is selected from the group consisting of F-SO₂R, CH₂F-SO₂R, CHF₂-SO₂R and CF₃-SO₂R, where R is a monovalent or divalent cation; preferably, R is an alkali metal cation, very preferably potassium.

8. Process for the preparation of a compound selected from the group consisting of a sulfonimide compound of formula (Ea-SO₂)₂NH and of its salts (Ea-SO₂)₂NM, a fluorinated sulfonic acid compound of formula Ea-SO₂OH and a fluorinated sulfonic anhydride compound of formula (Ea-SO₂)₂O, Ea having the definition specified in Claims 1 to 7 and M representing a metal, said process comprising:
- a stage of preparation of an oxysulfide and fluorinated compound according to the process described above in any one of Claims 1 to 7,
- a stage in which said oxysulfide and fluorinated compound is used as reactive compound in the synthesis of a sulfonimide compound of formula (Ea-SO₂)₂NH and of its salts (Ea-SO₂)₂NM, of a fluorinated sulfonic acid compound of formula Ea-SO₂OH or of a fluorinated sulfonic anhydride compound of formula (Ea-SO₂)₂O.

9. Process according to Claim 8, **characterized in that** the compound obtained is a sulfonimide compound or one of its salts selected from the group consisting of bis(trifluoromethanesulfonyl)imide of formula (CF₃SO₂)₂NH, lithium bis(trifluoromethanesulfonyl)imide of formula (CF₃SO₂)₂NLi, bis(fluorosulfonyl)imide of formula (F-SO₂)₂NH and lithium bis(fluorosulfonyl)imide of formula (F-SO₂)₂NLi.

10. Process according to Claim 8, **characterized in that** the compound obtained is a fluorinated sulfonic acid or a fluorinated sulfonic anhydride compound selected from the group consisting of trifluoromethanesulfonic acid of formula CF₃SO₂OH and trifluoromethanesulfonic anhydride of formula (CF₃SO₂)₂O.
